(19) Europäisches Patentamt
European Patent Office
Office européen des brevets

(11) Veröffentlichungsnummer : **0 251 083 B1**

(12) # EUROPÄISCHE PATENTSCHRIFT

(45) Veröffentlichungstag der Patentschrift : **30.01.91 Patentblatt 91/05**

(51) Int. Cl.[5] : **C07D 239/48,** A01N 43/54

(21) Anmeldenummer : **87108849.8**

(22) Anmeldetag : **20.06.87**

(54) **4-Aminopyrimidinderivate.**

(30) Priorität : **21.06.86 DE 3620841**

(43) Veröffentlichungstag der Anmeldung : **07.01.88 Patentblatt 88/01**

(45) Bekanntmachung des Hinweises auf die Patenterteilung : **30.01.91 Patentblatt 91/05**

(84) Benannte Vertragsstaaten : **AT BE CH DE ES FR GB GR IT LI NL SE**

(56) Entgegenhaltungen :
**BE-A- 518 622**
**FR-A- 1 572 620**
**CHEMICAL ABSTRACTS, Band 95, 1981, Seite 648, Zusammenfassung Nr. 97712f, Columbus, Ohio, US; D. GHOSH: "2,4-Bis(arylamino)-6-methylpyrimidines as antimicrobial agents"**

(73) Patentinhaber : **BASF Aktiengesellschaft**
**Carl-Bosch-Strasse 38**
**D-6700 Ludwigshafen (DE)**

(72) Erfinder : **Eicken, Karl, Dr.**
**Am Huettenwingert 12**
**D-6706 Wachenheim (DE)**
Erfinder : **Wahl, Peter, Dr**
**Valentinianstrasse 8**
**D-6802 Ladenburg (DE)**
Erfinder : **Ammermann, Eberhard, Dr.**
**Sachsenstrasse 3**
**D-6700 Ludwigshafen (DE)**
Erfinder : **Pommer, Ernst-Heinrich, Dr.**
**Berliner Platz 7**
**D-6703 Limburgerhof (DE)**

EP 0 251 083 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 4-Aminopyrimidinderivate der allgemeinen Formel I

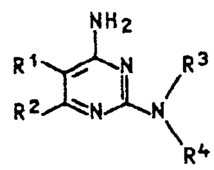

I

in der die Substituenten folgende Bedeutung haben

$R^1$ $C_1$-$C_{20}$-Alkyl, $C_2$-$C_{20}$-Alkoxy-alkyl,

$R^2$ Wasserstoff oder $C_1$-$C_{20}$-Alkyl,

$R^3$, $R^4$ $C_1$-$C_{20}$-Alkyl mit der Maßgabe, daß $R^1$ nicht $C_1$-$C_5$-Alkyl ist, wenn $R^3$ und $R^4$ für Methyl oder Ethyl stehen, $C_2$-$C_{20}$-Alkenyl oder

$R^3$ und $R^4$ bilden gemeinsam mit dem Stickstoffatom einen 5- oder 6-gliedrigen Ring, der Sauerstoff enthalten und mit $C_1$-$C_8$-Alkyl auch ein-oder mehrfach substituiert sein kann.

Außerdem betrifft die vorliegende Erfindung die Herstellung der Verbindungen I, die Verwendung der 4-Aminopyrimidinverbindungen I als Fungizide, sowie Fungizide, die die Verbindungen I enthalten und ein Verfahren zur Bekämpfung von Pilzen.

Aus der US-A-3 670 077 ist die Verbindung I'

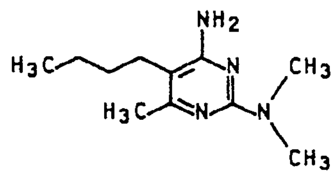

(I')

als Fungizid bekannt, deren Wirkung jedoch zu wünschen übrig läßt.

Der Erfindung lag daher die Aufgabe zugrunde, neue 4-Aminopyrimidinderivate mit im Vergleich zur Verbindung I' verbesserter fungizider Wirkung bereitzustellen.

Demgemäß wurden die eingangs definierten neuen 4-Aminopyrimidinderivate I sowie Verfahren zu deren Herstellung gefunden. Weiterhin wurde gefunden, daß sich die Verbindung I hervorragend als Fungizide eignen.

Die Verbindungen I sind nach folgenden Methoden erhältlich : Ausgehend von β-Carbonylverbindungen II, die im Fall der β-Ketoester analog Organic Synthesis Coll. Vol. 1, 248, 2nd Edition 1948, leicht erhältlich sind, werden diese.

a) mit Thioharnstoff und Alkoholaten, beispielsweise Alkalialkoholaten in Alkoholen in an sich bekannter Weise in die 2-Thiopyrimidinderivate III überführt,

$a_1$) diese dann in ebenfalls an sich bekannter Weise mit einer Verbindung $R^5$-X, in der $R^5$ einen niederen Alkylrest und X Chlor, Brom oder Jod bedeuten, oder einem Alkylsulfat $(R^5O)_2SO_2$ zu den 2-Thiopyrimidonderivaten IV umsetzt und

$a_2$) diese anschließend in ebenfalls an sich bekannter Weise mit einem Amin $HNR^3R^4$, vorzugsweise sekundäre cyclische Amine, zu den Pyrimidonderivaten V umsetzt oder

b) mit einem substituierten Guanidinderivat

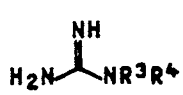

oder deren Salze in an sich bekannter Weise zu den Pyrimidonderivaten V umsetzt.

Die so erhaltenen Pyrimidonderivate V lassen sich mit Chlorierungsmitteln, vorzugsweise mit Phosphoroxychlorid, in an sich bekannter Weise in 4-Position chlorieren und anschließend in ebenfalls an sich bekannter Weise mit Ammoniak unter Druck zu den 4-Aminopyrimidinderivaten I überführen.

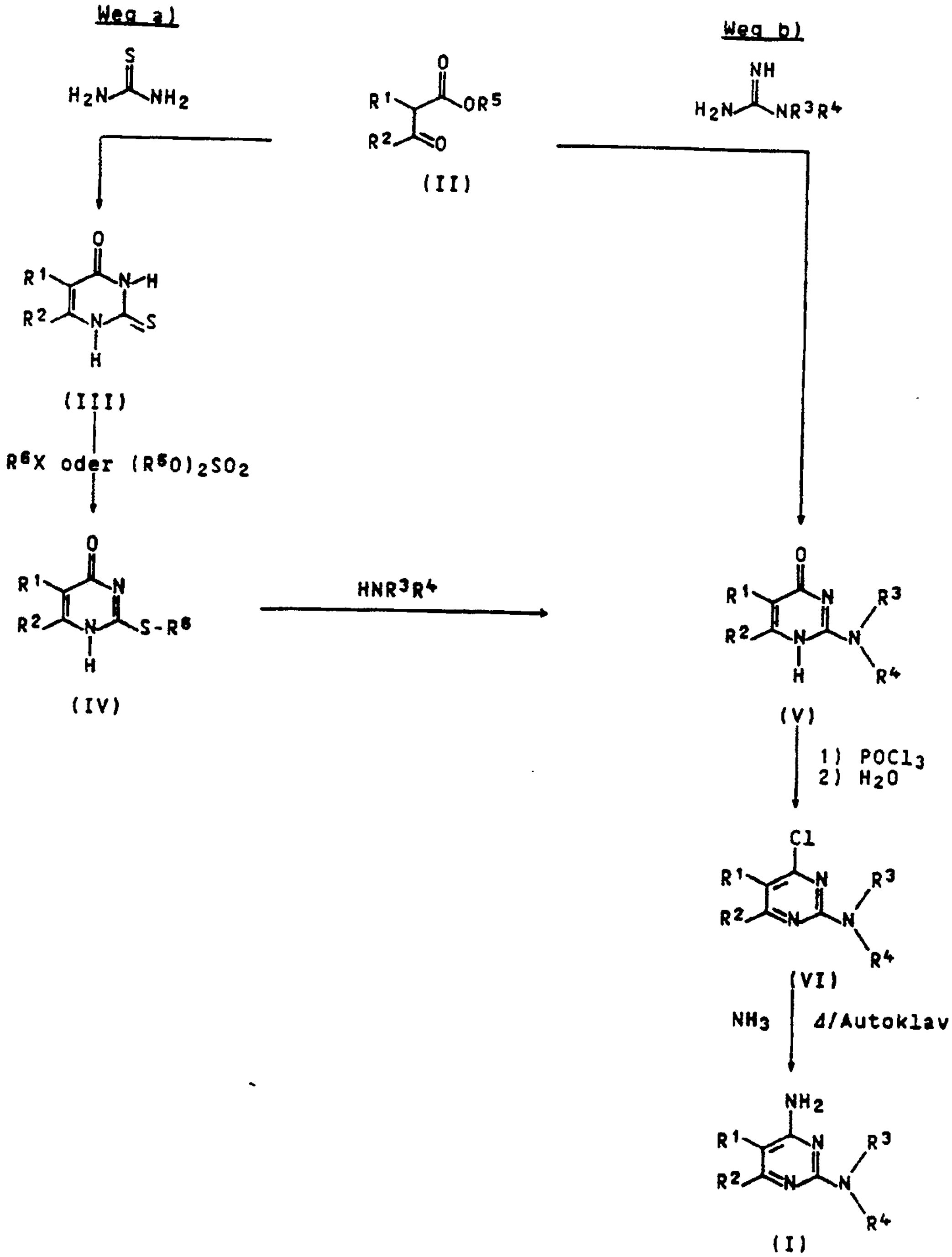


- Unter den Resten $R^1$ sind bevorzugt
  - $C_6$- bis $C_{18}$-Alkyl, besonders $C_8$- bis $C_{16}$-Alkyl, beispielsweise Octyl, Nonyl, Decyl, Undecyl, Dodecyl, Tetradecyl, Hexadecyl und deren Isomere
  - eine Alkylenkette mit 4 bis 5 C-Atomen, die zusammen mit dem Stickstoffatom Pyrrolidin bzw. Piperidin bedeuten,
  - $C_2$-$C_{20}$-Alkoxy-alkyl, besonders $C_6$- bis $C_{20}$-Alkoxy-alkyl, z.B 2-n-Hexyloxiethyl und 2-n-Octyloxiethyl,
- Unter den Resten $R^2$ sind Wasserstoff und $C_1$-bis $C_8$-Alkyl, besonders $C_1$-$C_4$-Alkyl, beispielsweise Methyl, Ethyl, iso-Propyl, bevorzugt.
- Unter der Voraussetzung, daß $R^1$ nicht $C_1$-bis $C_5$-Alkyl ist, wenn $R^3$ und $R^4$ für Methyl und Ethyl steht, sind unter den Resten $R^3$ und $R^4$ bevorzugt

– $C_1$- bis $C_{12}$-Alkyl, besonders $C_1$ bis $C_4$-Alkyl wie Methyl, Ethyl n-Propyl, iso-Propyl, n-Butyl, sec.-Butyl,

– $C_2$- bis $C_8$-Alkenyl, besonders $C_3$- und $C_4$-Alkenyl, beispielsweise Allyl und But-2-en-1-yl,

– eine Alkylenkette mit 4 bis 5 C-Atomen, die zusammen mit dem Stickstoffatom Pyrrolidin bzw. Piperidin bedeuten,

– eine durch Methyl oder Ethyl auch mehrfach substituierte Alkylenkette mit 4 bis 5 C-Atomen, die zusammen mit dem Stickstoffatom Pyrrolidin- bzw. Piperidinderivate bedeuten, wie beispielsweise 3-Methylpyrrolidin, 3,3-Dimethylpyrrolidin, 3-Ethylpyrrolidin, 2-Methyl-, 2-Ethylpiperidin, 3-Methyl-, 3-Ethylpiperidin, 4-Methyl-, 4-Ethylpiperidin,

– eine durch Sauerstoff unterbrochene Alkylenkette mit beispielsweise 4 C-Atomen wie Morpholin,

– eine durch Methyl oder Ethyl auch mehrfach substituierte Alkylenkette mit beispielsweise 4 C-Atomen wie cis-2,6-Dimethylmorpholin oder trans-2,6-Dimethylmorpholin, cis- oder trans-2,6-Diethylmorpholin.

Im Hinblick auf die bestimmungsgemäße Verwendung der 4-Aminopyrimidinderivate werden diejenigen Verbindungen I besonders bevorzugt, bei denen $R^1$ eine $C_8$-$C_9$-Alkylgruppe, $R^2$ die Methylgruppe, $R^3$ und $R^4$ unabhängig voneinander die Methyl- oder Ethylgruppe oder $R^3$ und $R^4$ zusammen mit dem gemeinsamen Stickstoffatom einen Pyrrolidin-, Piperidin-, Morpholin- oder Dimethylmorpholinrest bedeuten.

Beispiele für geeignete Verbindungen I sind

1. 2-(N,N-Dimethylamino)-4-amino-5-n-octyl-6-methylpyrimidin
2. 2-(N-Methyl-N-ethylamino)-4-amino-5-n-octyl-6-methylpyrimidin
3. 2-(N,N-Diethylamino)-4-amino-5-n-octyl-6-methylpyrimidin
4. 2-(N-Methyl-N-n-propylamino)-4-amino-5-n-octyl-6-methylpyrimidin
5. 2-(N-Methyl-N-n-butylamino)-4-amino-5-n-octyl-6-methylpyrimidin
6. 2-(N,N-Di-n-propylamino)-4-amino-5-n-octyl-6-methylpyrimidin
7. 2-(N,N-Di-n-butylamino)-4-amino-5-n-octyl-6-methylpyrimidin
8. 2-Pyrrolidino-4-amino-5-n-octyl-6-methylpyrimidin
9. 2-Piperidino-4-amino-5-n-octyl-6-methylpyrimidin
10. 2-(2′-Methylpiperidino)-4-amino-5-n-octyl-6-methylpyrimidin
11. 2-(3′-Methylpiperidino)-4-amino-5-n-octyl-6-methylpyrimidin
12. 2-(3′,3′-Dimethylpiperidino)-4-amino-5-n-octyl-6-methylpyrimidin
13. 2-Morpholino-4-amino-5-n-octyl-6-methylpyrimidin
14. 2-(trans-3′,5′-Dimethylmorpholino)-4-amino-5-n-octyl-6-methylpyrimidin
15. 2-(cis-3′,5′-Dimethylmorpholino)-4-amino-5-n-octyl-6-methylpyrimidin
16. 2-(N,N-Dimethylamino)-4-amino-5-n-nonyl-6-methylpyrimidin
17. 2-(N,N Diethylamino)-4-amino-5-n-nonyl-6-methylpyrimidin
18. 2-(N-Methyl-N-ethylamino)-4-amino-5-n-nonyl-6-methylpyrimidin
19. 2-(N,N-Di-n-propylamino)-4-amino-5-n-nonyl-6-methylpyrimidin
20. 2-(N,Methyl-N-allylamino)-4-amino-5-n-nonyl-6-methylpyrimidin
21. 2-(N-Ethyl-N-allylamino)-4-amino-5-n-nonyl-6-methylpyrimidin
22. 2-(N,N-Diallylamino)-4-amino-5-n-nonyl-6-methylpyrimidin
23. 2-Pyrrolidino-4-amino-5-n-nonyl-6-methylpyrimidin
24. 2-Piperidino-4-amino-5-n-nonyl-6-methylpyrimidin
25. 2-Morpholino-4-amino-5-n-nonyl-6-methylpyrimidin
26. 2-(N,N Dimethylamino)-4-amino-5-n-decyl-6-methylpyrimidin
27. 2-(N,N-Diethylamino)-4-amino-5-n-decyl-6-methylpyrimidin
28. 2-(N-Methyl-N-ethylamino)-4-amino-5-n-decyl-6-methylpyrimidin
29. 2-(N-Methyl-N-allylamino)-4-amino-5-n-decyl-6-methylpyrimidin
30. 2-Pyrrolidino-4-amino-5-n-decyl-6-methylpyrimidin
31. 2-Piperidino-4-amino-5-n-decyl-6-methylpyrimidin
32. 2-Morpholino-4-amino-5-n-decyl-6-methylpyrimidin
33. 2-(N,N-Dimethylamino)-4-amino-5-n-undecyl-6-methylpyrimidin
34. 2-(N,N-Diethylamino)-4-amino-5-n-undecyl-6-methylpyrimidin
35. 2-Pyrrolidino-4-amino-5-n-undecyl-6-methylpyrimidin
36. 2-(N,N-Dimethylamino)-4-amino-5-n-dodecyl-6-methylpyrimidin
37. 2-(N,N-Diethylamino)-4-amino-5-n-dodecyl-6-methylpyrimidin
38. 2-Pyrrolidino-4-amino-5-n-dodecyl-6-methylpyrimidin
39. 2-Piperidino-4-amino-5-n-dodecyl-6-methylpyrimidin
40. 2-Morpholino-4-amino-5-n-dodecyl-6-methylpyrimidin
41. 2-(N,N-Dimethylamino)-4-amino-5-(2-ethylhexyl)-6-methylpyrimidin
42. 2-(N,N-Diethylamino)-4-amino-5-(2-ethylhexyl)-6-methylpyrimidin

43. 2-Pyrrolidino-4-amino-5-(2-ethylhexyl)-6-methylpyrimidin
44. 2-Piperidino-4-amino-5-(2-ethylhexyl)-6-methylpyrimidin
45. 2-Morpholino-4-amino-5-(2-ethylhexyl)-6-methylpyrimidin
46. 2-(N,N-Dimethylamino)-4-amino-5-(3,5,5-trimethylpentyl)-6-methylpyrimidin
47. 2-(N-Methyl-N-ethylamino)-4-amino-5-(3,5,5-trimethylpentyl)-6-methylpyrimidin
48. 2-(N,N-Dimethylamino)-4-amino-5-(3,5,5-trimethylpentyl)-6-methylpyrimidin
49. 2-Pyrrolidino-4-amino-5-(3,5,5-trimethylpentyl)-6-methylpyrimidin
50. 2-Piperidino-4-amino-5-(3,5,5-trimethylpentyl)-6-methylpyrimidin
51. 2-Morpholino-4-amino-5-(3,5,5-trimethylpentyl)-6-methylpyrimidin
52. 2-(N,N-Dimethylamino)-4-amino-5-n-tetradecyl-6-methylpyrimidin
53. 2-(N,N-Diethylamino)-4-amino-5-n-tetradecyl-6-methylpyrimidin
54. 2-Pyrrolidino-4-amino-5-n-tetradecyl-6-methylpyrimidin
55. 2-(N,N-Dimethylamino)-4-amino-5-n-hexadecyl-6-methylpyrimidin
56. 2-(N,N-Diethylamino)-4-amino-5-n-hexadecyl-6-methylpyrimidin
57. 2-Pyrrolidino-4-amino-5-n-hexadecyl-6-methylpyrimidin

Die neuen Wirkstoffe zeigen eine starke fungitoxische Wirksamkeit gegen phytopathogene Pilze, insbesondere aus der Klasse der Phycomyceten. Sie sind daher zur Bekämpfung von beispielsweise Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben sowie von Erysiphe graminis an Getreide, Uncinula necator an Reben, Podosphaera leucotricha an Äpfeln und Pyrenophora teres an Gerste geeignet.

Die Wirkstoffe weisen eine hohe Pflanzenverträglichkeit auf. Ein Teil der Wirkstoffe zeigt kurative Eigenschaften, d.h. die Anwendung der Mittel kann noch nach der Pilzinfektion der Pflanzen durch die Krankheitserreger vorgenommen werden, um einen Bekämpfungserfolg zu erzielen.

Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 bis 5 kg Wirkstoff je ha.

Die Wirkstoffe können auch zusammen mit anderen Wirkstoffen z.B. Herbiziden, Insektiziden, Wachstumsregulatoren und anderen Fungiziden, oder auch mit Düngemitteln vermischt und ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise :

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramidsulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid ;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;
5-Nitro-isophthalsäure-di-isopropylester
heterocyclische Substanzen, wie
2-Heptadecyl-2-imidazolin-acetat,

2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-cabaminsäuremethylester,
2-Methoxycarbonylamino-benzimidazol,
2-(Furyl-(2))-benzimidazol,
2-(Thiazolyl-(4))-benzimidzol,
N-(1,1,2,2-Tetrachlorethylthio)-tetrahydrophthalimid,
N-Trichlormethylthio-tetrahydrophthalimid,
N-Trichlormehylthio-phthalimid,
N-Dichlorfluormethylthio-N',N'-dimethyl-N-phenyl-schwefelsäurediamid,
5-Ethoxy-3-trichlormethyl-1,2,4-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Metyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-bezoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-mopholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl)-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-et yl]-1H-1,2,4-triazol,
N-(n-Propyl)-N-(2,4,6-trichlorphenoxyethyl)-N'-imidazol-yl-harnstoff,
1-(4-Chlorphenoxy)-3,3 dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,

2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol.

Die neuen Wirkstoffe werden beispielsweise in Form von direkt versprühbaren Lösungen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken ; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten oder Öldispersionen aus Mineralölfraktionen von mittlerem bis hohem Siedepunkt, wie Kerosin oder Dieselöl, ferner Kohlenteerölen usw., sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten, oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfate, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolalkohol, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Attaclay, Kalkstein, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

## Herstellungbeispiele

### Methode A : Herstellung der 4-Aminopyrimidine I mit Thioharnstoff (Weg a)

#### Beispiel 1

13,4 g 2-Methylthio-4-oxo-5-n-octyl-6-methylpyrimidin (J. Pharm. Soc. Japan ; Vol. 96, 384 (1976)) und 7,5 ml Piperidin wurden in 50 ml Ethylglykol 14 Stunden unter Durchleiten eines schwachen Stickstoffstroms am Rückfluß erhitzt. Nach dem Abkühlen erhielt man durch Absaugen, Waschen mit wenig kaltem Methanol und Trocknen im Vakuum bei 60°C 12,5 g 2-Piperidino-4-oxo-5-n-octyl-6-methylpyrimidin vom Fp. 95°C.

39,2 g dieses Pyrimidons wurden in 120 ml Phosphoxychlorid 20 Minuten bei 60°C gerührt. Nach dem Entfernen des Phosphoxychlorids im Vakuum wurden zweimal je 60 ml Toluol zugegeben und jeweils im Vakuum verdampft. Der Rückstand wurde zwischen 300 ml $H_2O$ und 200 ml Methylenchlorid verteilt. Die wäßrige Phase wurde noch einmal mit 50 ml Methylenchlorid nachextrahiert. Die vereinigten organischen Phasen wurden eingeengt und mit Toluol an Kieselgel (200 g) chromatographische gereinigt. Nach dem Verdampfen des Toluols isolierte man 28,8 g 2-Piperidino-4-chlor-5-n-octyl-6-methylpyrimidin als farbloses Öl.

23,0 g dieser Chlorverbindung wurden in 90 ml Ethanol gelöst, 80 ml Ammoniak aufgepreßt und 24

Stunden bei 200°C im Autoklav gerührt. Der Austrag wurde nach dem Verdampfen des Ethanols zwischen 100 ml Wasser und 100 ml Ether verteilt und mit gesättigter Natriumbicarbonat-Lösung auf ph 7 bis 8 eingestellt. Aus der Etherphase isolierte man nach Trocknen, Verdampfen des Lösungsmittels und Umkristallisieren des Rückstands (18,7 g) aus 50 ml n-Pentan (Absaugen bei −15°C) 12,0 g 2-Piperidino-4-amino-5-n-octyl-6-methylpyrimidin (Verbindung 9) vom Fp. 55°C.

Methode B : Herstellung der 4-Aminopyrimidinderivate II mit Guanidinderivaten (Weg b)

Beispiel 2

Zu einer Lösung von 24,7 g 1,1-Dimethylguanidin-Hydrochlorid in 200 ml Methanol tropfte man bei 0°C 79,2 g 30%igen Natriummethylat-Lösung und anschließend 48,4 g 2-n-Nonylacetessigsäuremethylester zu. Man rührte 5 h bei 0°C und 12 Stunden bei Raumtemperatur nach. Nach dem Verdampfen des Methanols wurde der Rückstand in 1l Wasser gelöst und mit Eisessig auf pH 7 eingestellt. Nach dem Absaugen, Waschen mit kaltem Wasser und Methanol von 0°C (2 x 5 ml) erhielt man nach Trocknung im Vakuum 24,5 g 2-N-Dimethylamino-4-oxo-5-n-nonyl-6-methyl-pyrimidin vom Fp. 76°C.

24,5 g dieses Pyrimidons wurden in 100 ml Phosphoxychlorid 30 Minuten bei 60°C gerührt. Nach dem Entfernen des Phosphoxychlorids im Vakuum wurden zweimal je 30 ml Toluol zugegeben und jeweils im Vakuum verdampft. Der Rückstand wurde zwischen 300 ml Wasser und 200 ml Methylenchlorid verteilt. Die wäßrige Phase wurde einmal mit 50 ml Methylenchlorid nachextrahiert. Nach Trocknen und Eindampfen der vereinigten organischen Phasen isolierte man 22,0 g 2-N-Dimethylamino-4-chlor-5-n-nonyl-6-methylpyrimidin als Kristallbrei.

22,0 g dieses 4-Chlorpyrimidins wurden in 90 ml Ethanol gelöst, 80 ml Ammoniak aufgepreßt und 24 Stunden bei 200°C im Autoklav gerührt. Nach der Aufarbeitung wie in Beispiel 1c) isolierte man 15,5 g kristallines Rohprodukt, das nach dem Umkristallisieren aus 25 ml n-Pentan (Absaugen bei −15°C) 8,7 g 2-N-Dimethylamino-4-amino-5-n-nonyl-6-methylpyrimidin (Verbindung 16) vom Fp. 56 bis 57°C ergab.

Nach den oben beschriebenen Verfahren können beispielsweise folgende 4-Aminopyrimidine I hergestellt werden :

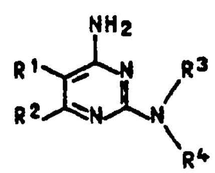

(I)

| Verbindung Nr. | $R^1$ | Methode | $R^2$ | $R^3$ | $R^4$ | Fp.[°C] |
|---|---|---|---|---|---|---|
| 1 | $n-C_8H_{17}$ | | $CH_3$ | $CH_3$ | $CH_3$ | 58- 61 |
| 2 | $n-C_8H_{17}$ | | $CH_3$ | $CH_3$ | $C_2H_5$ | Öl |
| 3 | $n-C_8H_{17}$ | | $CH_3$ | $C_2H_5$ | $C_2H_5$ | 43- 46 |
| 4 | $n-C_8H_{17}$ | | $CH_3$ | $CH_3$ | $n-C_3H_7$ | |
| 5 | $n-C_8H_{17}$ | | $CH_3$ | $CH_3$ | $n-C_4H_9$ | 35- 36 |
| 6 | $n-C_8H_{17}$ | | $CH_3$ | $n-C_3H_7$ | $n-C_3H_7$ | 29 |
| 7 | $n-C_8H_{17}$ | | $CH_3$ | $n-C_4H_9$ | $n-C_4H_9$ | Öl |
| 8 | $n-C_8H_{17}$ | | $CH_3$ | $-(CH_2)_4-$ | | 75- 78 |
| 9 | $n-C_8H_{17}$ | | $CH_3$ | $-(CH_2)_5-$ | | 55 |
| 10 | $n-C_8H_{17}$ | | $CH_3$ | $-CH(CH_3)-(CH_2)_4$ | | |
| 11 | $n-C_8H_{17}$ | | $CH_3$ | $-CH_2-CH(CH_3)-(CH_2)_3-$ | | 57- 58 |
| 12 | $n-C_8H_{17}$ | | $CH_3$ | $-CH_2-C(CH_3)_2-(CH_2)_3-$ | | 71 |
| 13 | $n-C_8H_{17}$ | | $CH_3$ | $-(CH_2)_2-O-(CH_2)_2$ | | 50- 51 |
| 14 | $n-C_8H_{17}$ | | $CH_3$ | $-CH_2-CH(CH_3)-O-CH(CH_3)-CH_2-$ (trans) | | 48- 59 |
| 15 | $n-C_8H_{17}$ | | $CH_3$ | $-CH_2-CH(CH_3)-O-CH(CH_3)-CH_2-$ (cis) | | 56- 59 |
| 16 | $n-C_9H_{19}$ | | $CH_3$ | $CH_3$ | $CH_3$ | 56- 57 |
| 17 | $n-C_9H_{19}$ | | $CH_3$ | $C_2H_5$ | $C_2H_5$ | 35- 36 |
| 18 | $n-C_9H_{19}$ | | $CH_3$ | $CH_3$ | $C_2H_5$ | |
| 19 | $n-C_9H_{19}$ | | $CH_3$ | $n-C_3H_7$ | $n-C_3H_7$ | |
| 20 | $n-C_9H_{19}$ | | $CH_3$ | $CH_3$ | $CH_2-CH=CH_2$ | |
| 21 | $n-C_9H_{19}$ | | $CH_3$ | $C_2H_5$ | $CH_2-CH=CH_2$ | |
| 22 | $n-C_9H_{19}$ | | $CH_3$ | $CH_2-CH=CH_2$ | $CH_2-CH=CH_2$ | |

| Verbindung Nr. | $R^1$ | Methode | $R^2$ | $R^3$ | $R^4$ | Fp.[°C] |
|---|---|---|---|---|---|---|
| 23 | $n-C_9H_{19}$ | | $CH_3$ | | $-(CH_2)_4-$ | 91 · 92 |
| 24 | $n-C_9H_{19}$ | | $CH_3$ | | $-(CH_2)_5-$ | 60 · 61 |
| 25 | $n-C_9H_{19}$ | | $CH_3$ | | $-(CH_2)_2-O-(CH_2)_2-$ | 46 - 47 |
| 26 | $n-C_{10}H_{21}$ | | $CH_3$ | $CH_3$ | $CH_3$ | 45 |
| 27 | $n-C_{10}H_{21}$ | | $CH_3$ | $C_2H_5$ | $C_2H_5$ | Öl |
| 28 | $n-C_{10}H_{21}$ | | $CH_3$ | $CH_3$ | $C_2H_5$ | |
| 29 | $n-C_{10}H_{21}$ | | $CH_3$ | $CH_3$ | $CH_2-CH=CH_2$ | |
| 30 | $n-C_{10}H_{21}$ | | $CH_3$ | | $-(CH_2)_4-$ | 83-84 |
| 31 | $n-C_{10}H_{21}$ | | $CH_3$ | | $-(CH_2)_5-$ | 34-35 |
| 32 | $n-C_{10}H_{21}$ | | $CH_3$ | | $-(CH_2)_2-O-(CH_2)_2$ | |
| 33 | $n-C_{11}H_{23}$ | | $CH_3$ | $CH_3$ | $CH_3$ | |
| 34 | $n-C_{11}H_{23}$ | | $CH_3$ | $C_2H_5$ | $C_2H_5$ | |
| 35 | $n-C_{11}H_{23}$ | | $CH_3$ | | $-(CH_2)_4-$ | |
| 36 | $n-C_{12}H_{25}$ | | $CH_3$ | $CH_3$ | $CH_3$ | |
| 37 | $n-C_{12}H_{25}$ | | $CH_3$ | $C_2H_5$ | $C_2H_5$ | |
| 38 | $n-C_{12}H_{25}$ | | $CH_3$ | | $-(CH_2)_4-$ | |
| 39 | $n-C_{12}H_{25}$ | | $CH_3$ | | $-(CH_2)_5-$ | |
| 40 | $n-C_{12}H_{25}$ | | $CH_3$ | | $-(CH_2)_2-O-(CH_2)_2-$ | |
| 41 | $n-C_4H_9-CH(C_2H_5)-CH_2$ | | $CH_3$ | $CH_3$ | $CH_3$ | Öl |
| 42 | $n-C_4H_9-CH(C_2H_5)-CH_2$ | | $CH_3$ | $C_2H_5$ | $C_2H_5$ | |
| 43 | $n-C_4H_9-CH(C_2H_5)-CH_2$ | | $CH_3$ | | $-(CH_2)_4-$ | 86 |
| 44 | $n-C_4H_9-CH(C_2H_5)-CH_3$ | | $CH_3$ | | $-(CH_2)_5-$ | |
| 45 | $n-C_4H_9-CH(C_2H_5)-CH_2$ | | $CH_3$ | | $-(CH_2)_2-O-(CH_2)_2-$ | |

| Verbindung Nr. | $R^1$ | Methode | $R^2$ | $R^3$ | $R^4$ | Fp.[°C] |
|---|---|---|---|---|---|---|
| 46 | $(CH_3)_3C-CH_2-CH(CH_3)-(CH_2)_2$ | | $CH_3$ | $CH_3$ | $CH_3$ | 77 |
| 47 | $(CH_3)_3C-CH_2-CH(CH_3)-(CH_2)_2$ | | $CH_3$ | $CH_3$ | $C_2H_5$ | |
| 48 | $(CH_3)_3C-CH_2-CH(CH_3)-(CH_2)_2$ | | $CH_3$ | $C_2H_5$ | $C_2H_5$ | 54-56 |
| 49 | $(CH_3)_3C-CH_2-CH(CH_3)-(CH_2)_2$ | | $CH_3$ | $-(CH_2)_4-$ | | 100-101 |
| 50 | $(CH_3)_3C-CH_2-CH(CH_3)-(CH_2)_2$ | | $CH_3$ | $(CH_2)_5-$ | | 69-70 |
| 51 | $(CH_3)_3C-CH_2-CH(CH_3)-(CH_2)_2$ | | $CH_3$ | $-(CH_2)_2-O-(CH_2)_2$ | | |
| 52 | $n\text{-}C_{14}H_{29}$ | | $CH_3$ | $CH_3$ | $CH_3$ | |
| 53 | $n\ C_{14}H_{29}$ | | $CH_3$ | $C_2H_5$ | $C_2H_5$ | |
| 54 | $n\text{-}C_{14}H_{29}$ | | $CH_3$ | $-(CH_2)_4-$ | | |
| 55 | $n\text{-}C_{14}H_{29}$ | | $CH_3$ | $CH_3$ | $CH_3$ | |
| 56 | $n\text{-}C_{16}H_{33}$ | | $CH_3$ | $C_2H_5$ | $C_2H_5$ | |
| 57 | $n\text{-}C_{16}H_{33}$ | | $CH_3$ | $-(CH_2)_4-$ | | |

Zubereitungsbeispiele für Pflanzenschutzmittel :

I. Man vermischte 90 Gew.-Teile der Verbindung des Beispiels 1 mit 100 Gew.-Teilen N-Methylpyrrolidon und erhielt eine Lösung, die zur Anwendung in Form kleinster Tropfen geeignet war.

II. 20 Gew.-Teile der Verbindung des Beispiels 2 wurden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl bestand. Durch Ausgießen und feines Verteilen der Lösung in Wasser erhielt man eine wäßrige Dispersion.

III. 20 Gew.-Teile der nach Beispiel 1 erhältlichen Verbindung wurden in einer Mischung gelöst, die aus 30 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl bestand. Durch Eingießen und Verrühren der Lösung in Wasser erhielt man eine wäßrige Dispersion.

IV. 20 Gew.-Teile der nach Beispiel 2 erhältlichen Verbindung wurden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl bestand. Durch Eingießen und Verrühren der Lösung in Wasser erhielt man eine wäßrige Dispersion.

V. 20 Gew.-Teile der nach Beispiel 1 erhältlichen Verbindung wurden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in Wasser erhielt man eine Spritzbrühe.

VI. 5 Gew.-Teile der nach Beispiel 2 erhältlichen Verbindung wurden mit 95 Gew.-Teilen feinteiligem Kaolin innig vermischt. Man erhielt auf diese Weise ein Stäubemittel, das 5 Gew.% des Wirkstoffs enthielt.

VII. 30 Gew.-Teile der nach Beispiel 1 erhältlichen Verbindung wurden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhielt auf diese Weise eine Aufbereitung des Wirkstoffs mit guter Haftfähigkeit.

VIII.40 Gew.-Teile der nach Beispiel 2 erhältlichen Verbindung wurden mit 30 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhielt eine stabile wäßrige Dispersion.

IX. 20 Gew.-Teile der nach Beispiel 1 erhältlichen Verbindung wurden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhielt eine stabile ölige Dispersion.

## Anwendungsbeispiele

### Beispiel A

Wirksamkeit gegen Phytophthora infestans an Tomaten

Blätter von Topfpflanzen der Sorte "Große Fleischtomate" wurden mit wäßriger Spritzbrühe, die 80 Gew.% Wirkstoff und 20 Gew.% Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18°C aufgestellt. Nach 6 Tagen hatte sich die Krankheit auf den unbehandelten, jedoch infizierten Kontrollpflanzen so stark entwickelt, daß die fungizide Wirksamkeit der Substanzen beurteilt werden konnte.

Das Ergebnis des Versuches zeigte, daß bei der Behandlung mit einer 0,025%igen Spritzbrühe die fungizide Wirksamkeit der Verbindungen 5, 11, 12, 14, 15 und 24 mit 85%, der Verbindungen 13, 16, 17, 23, 25 und 46 mit 95% und der Verbindungen 1, 2 und 8 mit 100% bedeutend höher ist, als bei der Vergleichsverbindung I' mit 10%.

Beispiel B

Wirksamkeit gegen Plasmopara viticola

Blätter von Topfreben der Sorte "Müller-Thurgau" wurden mit einer wäßrigen Spritzbrühe, die 80 Gew.% Wirkstoff und 20 Gew.% Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Reben-peronospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis des Versuches zeigte, daß bei der Behandlung mit einer 0,05%igen Spritzbrühe die fungizide Wirksamkeit der Verbindungen 2, 5, 11, 13 und 14 mit 85%, der Verbindungen 3, 12, 25 und 48 mit 95% und der Verbindungen 1, 8, 9 und 46 auf 100% wesentlich größer ist als bei der Vergleichsverbindung I' mit 50%.

## Ansprüche

1. 4-Aminopyrimidinderivate der allgemeinen Formel I

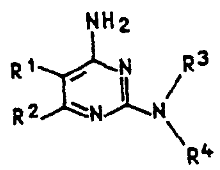

I

in der die Substituenten folgende Bedeutung haben

$R^1$ $C_1$-$C_{20}$-Alkyl, $C_2$-$C_{20}$-Alkoxy-alkyl,

$R^2$ Wasserstoff oder $C_1$-$C_{20}$-Alkyl,

$R^3$, $R^4$ $C_1$-$C_{20}$-Alkyl mit der Maßgabe, daß $R^1$ nicht $C_1$-$C_5$-Alkyl ist, wenn $R^3$ und $R^4$ für Methyl oder Ethyl stehen, $C_2$-$C_{20}$-Alkenyl oder

$R^3$ und $R^4$ bilden gemeinsam mit dem Stickstoffatom einen 5- oder 6-gliedrigen Ring, der Sauerstoff enthalten und mit $C_1$-$C_8$-Alkyl auch ein- oder mehrfach substituiert sein kann.

2. Verfahren zur Herstellung von 4-Aminopyrimidinderivaten I, dadurch gekennzeichnet, daß man β-Carbonylesterverbindungen der Formel II

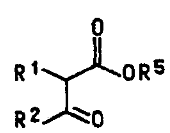

II

in der $R^5$ einen niederen Alkylrest bedeutet,

a) mit Thioharnstoff und einem Alkoholat in an sich bekannter Weise zu den 2-Thiopyrimidinderivaten III

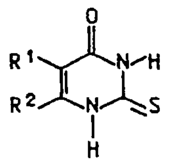

III

umsetzt, diese

$a_1$) in an sich bekannter Weise mit einer Verbindung $R^6$-X, in der $R^6$ einen niederen Alkylrest und X Chlor, Brom oder Jod bedeutet, oder einem Alkylsulfat $(R^6O)_2SO_2$ zu den 2-Thiopyrimidonderivaten IV

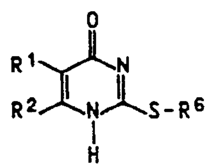

IV

umsetzt und diese Verbindungen IV

$a_2$) in an sich bekannter Weise mit einem Amin $HNR^3R^4$ zu den Pyrimidonderivaten V

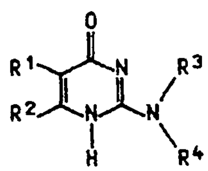

V.

umsetzt, oder daß man II

b) mit einem substituierten Guanidinderivat

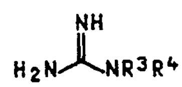

oder dessen Salzen in an sich bekannter Weise zu den Pyrimidinderivaten V umsetzt, und die Verbindungen V in an sich bekannter Weise in 4-Position chloriert und anschließend in ebenfalls an sich bekannter Weise in die Aminoverbindungen I überführt.

3. Verwendung eines 4-Aminopyrimidinderivates der Formel I gemäß Anspruch 1 als Fungizid.

4. Fungizid, enthaltend einen inerten Zusatzstoff und ein 4-Aminopyrimidinderivat der allgemeinen Formel I gemäß Anspruch 1.

5. Verfahren zur Bekämpfung von Pilzen, dadurch gekennzeichnet, daß man die Pilze oder die vor Pilzbefall zu schützenden Pflanzen, Saatgüter und Böden mit einer fungizid wirksamen Menge eines 4-Aminopyrimidinderivates der Formel I gemäß Anspruch 1 behandelt.

6. 4-Aminopyrimidinderivat der Formel I gemäß Anspruch 1, dadurch gekennzeichnet, daß $R^1$ $C_8$-$C_9$-Alkyl, $R^2$ Methyl, $R^3$ Methyl oder Ethyl, $R^4$ Methyl oder Ethyl oder $R^3$ + $R^4$ zusammen mit dem Stickstoffatom, dessen Substituenten sie sind, einem Pyrrolidin-, Piperidin-, Morpholin- oder Dimethylmorpholinrest bedeuten.

**Claims**

1. A 4-aminopyrimidine derivative of the formula I

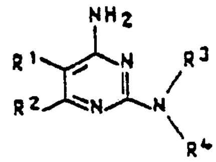

I

where $R^1$ is $C_1$-$C_{20}$-alkyl or $C_2$-$C_{20}$-alkoxyalkyl, $R^2$ is hydrogen or $C_1$-$C_{20}$-alkyl and $R^3$ and $R^4$ are each $C_1$-$C_{20}$-alkyl, with the proviso that $R^1$ is not $C_1$-$C_5$-alkyl when $R^3$ and $R^4$ are each methyl or ethyl, or $R^3$ and $R^4$ are each $C_2$-$C_{20}$-alkenyl, or $R^3$ and $R^4$, together with the nitrogen atom, form a 5-membered or 6 -membered ring which may contain oxygen and may furthermore be monosubstituted or polysubstituted by $C_1$-$C_8$-alkyl.

2. A process for the preparation of a 4-aminopyrimidine derivative I, wherein a β-carbonyl ester compound of the formula II

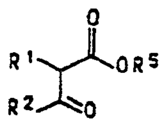

II

where $R^5$ is lower alkyl,

a) is reacted with thiourea and an alcoholate in a conventional manner to give a 2-thiopyrimidine derivative III

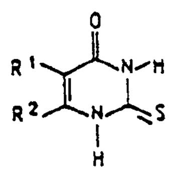

III

and the latter

$a_1$) is reacted in a conventional manner with a compound $R^6$-X, where $R^6$ is lower alkyl and X is chlorine, bromine or iodine, or with an alkyl sulfate $(R^6O)_2SO_2$ to give a 2 -thiopyrimidone derivative IV

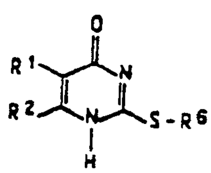

IV

and this compound IV

$a_2$) is reacted in a conventional manner with an amine $HNR^3R^4$ to give a pyrimidone derivative V

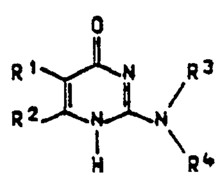

V

or II

b) is reacted with a substituted guanidine derivative

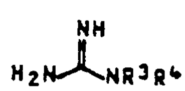

or its salts in a conventional manner to give a pyrimidine derivative V, and the compound V is chlorinated in the 4-position in a conventional manner and then converted into an amino compound I, likewise in a conventional manner.

3. Use of a 4-aminopyrimidine derivative of the formula I as claimed in claim 1 as a fungicide.

4. A fungicide containing an inert additive and a 4-aminopyrimidine derivative of the formula I as claimed in claim 1.

5. A method for controlling fungi, wherein the fungi or the plants, seeds and soils to be protected from fungal attack are treated with a fungicidal amount of a 4-aminopyrimidine derivative of the formula I as claimed in claim 1.

6. A 4-aminopyrimidine derivative of the formula I as claimed in claim 1, wherein $R^1$ is $C_8$- or $C_9$-alkyl, $R^2$ is methyl and $R^3$ and $R^4$ are each methyl or ethyl or $R^3$ and $R^4$, together with the nitrogen atom of which they are substituents, form a pyrrolidine, piperidine, morpholine or dimethylmorpholine radical.

## Revendications

1. Dérivé de 4-aminopyrimidine de la formule générale I

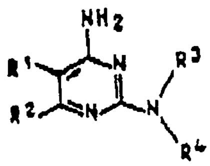

I

dans laquelle les substituants ont la signification suivante

$R^1$, alkyle en $C_1$-$C_{20}$, alcoxy-alkyle en $C_2$-$C_{20}$

$R^2$, hydrogène ou alkyle en $C_1$-$C_{20}$

$R^3$, $R^4$, alkyle en $C_1$-$C_{20}$, à condition que $R^1$ ne soit pas alkyle en $C_1$-$C_5$ quand $R^3$ et $R^4$ sont mis pour méthyle ou éthyle, alcényle en $C_2$-$C_{20}$, ou $R^3$ et $R^4$ forment ensemble avec l'atome d'azote un noyau à 5 ou 6 chaînons qui peut contenir de l'oxygène et peut être substitué aussi une ou plusieurs fois avec alkyle en $C_1$-$C_8$.

2. Procédé de préparation de dérivés de 4-aminopyrimidine I, caractérisé par le fait qu'on fait réagir des composés d'esters β-carbonyles de la formule II

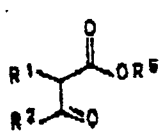

II

dans laquelle $R^5$ représente un reste alkyle inférieur

a) avec une thiourée et un alcoolat, de manière connue en soi, pour obtenir les dérivés de 2-thiopyrimidine III

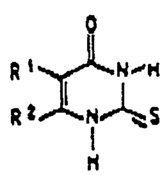

III

$a_1$) on fait réagir ceux-ci, de manière connue en soi, avec un composé $R^6$-X, où $R^6$ représente un reste alkyle inférieur et X, chlore, brome ou iode ou avec un sulfate d'alkyle $(R^6O)_2SO_2$, pour obtenir des dérivés de 2-thiopyrimidine IV

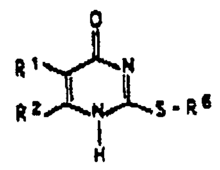

IV

$a_2$) et on fait réagir ces composés IV, de manière connue en soi, avec une amine $HNR^3R^4$, pour obtenir des dérivés de pyrimidone V

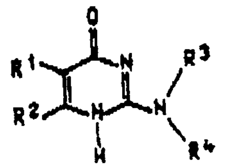

V

ou bien

b) on fait réagir, de manière connue en soi, le composé II avec un dérivé de guanidine substitué

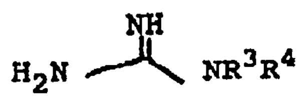

ou ses sels, pour obtenir les dérivés de pyrimidine V et on chlore en position-4, de manière connue en soi, les composés V et ensuite on les transforme, de manière également connue en soi, en les composés amino I.

3. Utilisation d'un dérivé de 4-aminopyrimidine de la formule I selon la revendication 1, comme fongicide,

4. Fongicide contenant un additif inerte et un dérivé de 4-aminopyrimidine de la formule I selon la revendication 1.

5. Procédé pour lutter contre les champignons, caractérisé par le fait qu'on traite les champignons ou les plantes, semences et, sols à protéger contre une attaque par les champigncns d'une quantité efficace du point de vue fongicide d'un dérivé de 4-aminopyrimidine de la formule I selon la revendication 1.

6. Dérivé de 4-aminopyrimidine de la formule I selon la revendication 1, caractérisé par le fait que $R^1$ représente alkyle en $C_8$-$C_9$, $R^2$, méthyle, $R^3$ méthyle ou éthyle, $R^4$ méthyle ou éthyle ou $R^3$ + $R^4$ ensemble avec l'atome d'azote, dont ils sont des substituants, un reste pyrrolidine, pipéridine, morpholine ou diméthylmorpholine.